# EUROPEAN PATENT APPLICATION

(11) **EP 4 589 284 A1**
(43) Date of publication of application: **23.07.2025**
(21) Application number: 24153160.7
(22) Date of filing: 22.01.2024
(51) Int. Cl.: G01N 21/41, G01N 21/47, G01N 21/77, C12Q 1/02, C12M 1/34, G01N 15/06, G01N 21/94, G01N 21/17, G01N 21/85, G01N 15/01

(54) **DEVICES, SYSTEMS AND METHODS FOR DETECTING MICROBIAL ACTIVITY**

(71) Applicant: The Procter & Gamble Company, Cincinnati, OH 45202 (US)
(72) Inventor: MENDEZ-MATA, Lucia, Newcastle upon Tyne, NE129BZ (GB); WRIGHT, Kevin Ian Trevor, Reading, RG20QE (GB); HENN, Christoph Florian, 41540 Dormagen (DE); Fraser-Kraus, Thomas, Heslington, York, YO105DD (GB); JOHNSON, Steven David, Heslington, York, YO10 5DD (GB); MARSDEN, Philipp Andrew, London, N17SL (GB)
(74) Representative: Gill Jennings & Every LLP

(57) **Abstract**

Disclosed herein are devices and systems for detecting microbial activity. A device (100) for detecting microbial contamination comprises a light source (20) that is configured to be optically coupled to an optical element (10), the optical element comprising a waveguide (2) and a diffraction grating (4), that together support a plurality of guided mode resonances at selected wavelengths of light from the light source. The device also comprises a condensing wall (40) having a condensing surface (50) on which liquid in gas (vapour) can condense, wherein the condensing surface is shaped, or engineered, to support flow of condensed liquids under gravity towards a collecting point (55) where the liquid can pool (6) around the optical element. The device further comprises a detector (30) configured to detect a property of a resonant guided mode of the plurality of resonant guided modes thereby to detect the presence or absence of microbes in the liquid that pools around the optical element.

## Description

### FIELD OF THE INVENTION

The present invention relates to devices, systems and methods for detecting the presence and activity of microbes using guided mode resonance using photonic guided mode resonances.

### BACKGROUND

Liquids, gases, or other materials held in enclosed environments, such as storage tanks and transport containers, are often susceptible to contamination if microbes or bacteria are present in them. This is especially undesirable in scenarios where healthcare or food products are stored potentially resulting in illness, or to a lesser extent material quality issues and waste.

Monitoring contamination in enclosed environments can be difficult. This is because taking manual samples to assess material and process hygiene can be logistically challenging due to difficulties in accessing these environments. Furthermore, manual sampling is an invasive technique that can, in itself, increase the risk of causing a contamination event. The materials held within storage tanks and transport containers may also be hazardous to human health, meaning that it may be preferable to avoid taking manual hygiene samples.

Some techniques currently used to measure microbial growth are based on the use of photonic sensors based on guided mode resonances. These constitute highly sensitive optical sensors that can accurately measure the refractive index of any chemistry or changes in refractive index associated with microbial growth present on the surface of the sensor via their attachment. In order to work effectively, these sensors generally need to be brought into contact with the test fluid.

For example, patent documents US 10753868 A1 and EP 3472593 A1 disclose a technique that involves using a narrowband light source beam to illuminate a chirped diffraction grating. The coupling position of the beam on the chirped grating is dependent on the refractive index of a sample disposed on the grating. By directly imaging the position of light (a guided mode resonance) reflected from the grating, the refractive index of the sample can be inferred. A change in the refractive index of the sample will accordingly change the coupling position of the beam on the grating. This will also therefore change the observed position on the grating from which the reflected resonating light originates.

Patent document PCT/GB2023/050748 discloses on optical sensor comprising a chirped diffraction grating that can be used to measure the refractive index of a fluid disposed thereon.

There is therefore a need to improve the measurement of the hygiene of process and stored materials and where this is compromised, detect the presence of microbial growth within enclosed environments, without the need for intrusive sampling.

### SUMMARY OF INVENTION

In an aspect of the present invention there is provided a device for detecting microbial activity, comprising: a light source that is configured to be optically coupled to an optical element, the optical element comprising a waveguide and a diffraction grating, that together support a plurality of guided mode resonances at selected wavelengths of light from the light source; a condensing wall having a condensing surface on which liquid in gas can condense, wherein the condensing surface is shaped to support the flow of condensed liquids under gravity towards a collecting point where the liquid can pool around the optical element; and a detector configured to detect a property of a resonant guided mode of the plurality of resonant guided modes, or otherwise resonating light, thereby to detect the presence or absence of microbes in the liquid that pools around the optical element.

More specifically, there is provided a device for detecting the presence and activity of microbes relative to a background level associated with the stored material or its dilution in condensate, comprising: a light source that is configured to be optically coupled to an optical element, the optical element comprising a waveguide and a diffraction grating, that together supports a plurality of guided mode resonances that may be resonant at the selected wavelengths of light from the light source; a condensing wall having a condensing surface on which liquid suspended in a gaseous carrier, such as air, can condense, wherein the condensing surface is shaped to support a flow and accumulation, or collection, of condensate under gravity towards a collecting point where the liquid can pool around the optical element; and a detector configured to detect the wavelength or the position of a resonant guided mode of the plurality of resonant guided modes thereby detecting the presence or absence of microbes in the liquid that pools around the optical element. As described herein, the term resonating light is the light of a resonant guided mode of the plurality of resonant guided modes.

In humid environments, such as storage tanks, condensate liquids can form, thereby creating a cycle in which material evaporates, condenses, and then flows back into the stored liquid. In practice, it is extremely difficult to sterilise or sanitize an enclosed environment where access is limited. Microbes can develop in these environments, and microbes can be present both in the stored liquid and in the associated condensate. In these circumstances, it has been found that it is possible to observe microbial growth in the condensed liquid at the top of a vessel or tank. Alternatively, moisture present in the vapour can condense into droplets due to temperature changes within the storage vessel. These droplets may carry chemistry and microbes from the bulk material or walls of the container to other regions of the container. Droplets can then form on various surfaces of the container, such as the ceiling, on which microbial growth can occur.

With the device of the present invention, liquid in the vapour can condense on the condensing surface if the device is installed at the top or on the sides of a vessel or tank. Attached and metabolising microbes within the condensed liquid can then be detected by the device relative to any dilute material chemistry present in the condensate. Furthermore, due to the high sensitivity of the guided mode resonance sensors, the device can detect chemical changes in biofilm as a consequence of microbial growth. The present invention can therefore function as an early warning microbial detection device. It is also possible that microbes may develop more rapidly on the surface of a bulk material in a storage tank, and therefore also in the condensed liquid. For this reason, detecting microbes in the condensed liquid may offer a technique for early detection, in comparison to techniques that focus on detection within the bulk liquid.

Additionally, the need for human-led hygiene sampling is negated by the present invention as the device can be installed in storage tanks or transport containers prior to their use. For example, the device can be installed and monitored, permanently or temporarily, in a storage or carrier vessel during its usage cycle, whether empty, being filled, stored, emptied or cleaned and sanitized.

When surface deposits are present on the diffraction grating of the optical element, the resonant wavelength of the resonating light of the shifts to a different wavelength, or the position of the resonating light on the diffraction grating may shift to a different position. These shifts are recorded by the detector, and their magnitudes may be indicative of the number of microbes or the metabolic activity of the microbes, or the change in viscosity of the biofilm deposited on the optical element.

By frequently recording the wavelength or the position of the resonant guided mode, or the resonating light, using the detector, the device can continuously monitor for any microbial activity in a particular environment in real time.

The condensing surface is shaped to support a flow of condensate under gravity towards a collecting point where the liquid can pool around the optical element. For example, the condensing surface may be curved, or angled, to support a flow of condensed liquids under gravity towards the collecting point. This is important because the optical sensor is designed to support guided mode resonance and detect microbial activity only when it is in contact with a liquid medium. Typically when condensation is formed it forms droplets, but the location of their formation is random, and therefore there is a need to locate the sensor in an area where the droplets can be made to aggregate on the sensor where microbial contamination can be detected. By flowing the condensate towards a collection point, it is easier to create a body of liquid that is large enough to coat the sensor. This pooling makes it easier to create the conditions that enable the sensing function of the guided mode resonances in the optical sensor to detect the composition of the fluid, and the detection of shifts in the wavelength or the position of the resonant light associated with the changes in refractive index associated with microbial contamination. It will be appreciated that a change in the resonant wavelength can be indicative of a change in the refractive index of the liquid medium, which, in turn, can be indicative of a change in condensate chemistry, due to the presence of microbes.

In various embodiments, the light source may be a narrowband light source and the diffraction grating may be a chirped diffraction grating. In such embodiments, the detector may be configured to detect a position of the resonating light. The coupling position of the narrowband light on a chirped diffraction grating is dependent on the refractive index of the liquid on the optical element. By detecting the position of the resonating light on the grating, the refractive index of the liquid can be inferred. A change in the refractive index of the liquid due to microbial growth will accordingly change the position on the grating at which the light is observed to resonates.

In various alternative embodiments, the light source may be a tuneable or broadband light source and the diffraction grating may be a traditional, unchirped diffraction grating. In such embodiments, the detector may be configured to detect a wavelength of the resonating light. The wavelength of the resonating light in a traditional, unchirped diffraction grating is dependent on the refractive index of the liquid on the optical element. By detecting the wavelength of the resonating light on the grating, the refractive index of the liquid can be inferred. A change in the refractive index of the liquid due to microbial growth will accordingly change the wavelength at which the light resonates.

In various embodiments, the optical element may be disposed on the collecting point. This can allow the condensed liquid to flow towards a gravitational minimum at the collecting point so that it can pool effectively on the diffraction grating. This can maximise the effectiveness of the detector in identifying changes in the liquid chemistry, even if the number of condensed droplets is small. In alternative embodiments, the diffraction grating may be disposed on the condensing surface at a location different to that of the collecting point, especially where the volume of condensate produced ensures all exposed surfaces above the bulk material are coated with fluid. The condensed liquid may pool on the diffraction grating of the optical element.

Preferably, the condensing surface includes a lower portion where the collecting point is located. In this way, the condensed liquids can flow downwards, along a flow path on the condensing surface, under gravity, and onto the collecting point. Any microbial growth can then occur around or on the collecting point and can be detected. Other substances present within the condensed liquids can also accumulate at this point.

Preferably, the condensing surface has a domed shape. It has been found that this is a suitable shape that promotes condensation on the surface and supports a flow of condensed liquid towards the bottom of the dome. In various embodiments, the condensing wall comprises glass. The interior surface of the condensing wall, which may be referred to as an illumination surface herein, may also have a domed shape. In various embodiments, the condensing surface may be engineered to enable the collection of condensates. For example, the material that the condensing surface is comprised of may comprise a particular surface energy, topography, and/or hydrophobicity that encourages the collection of droplets of condensate liquids formed thereon.

Preferably, the diffraction grating, or the optical element, is disposed at the apex of the dome shaped condensing surface. In this way, the condensate liquids can flow downwards along the condensing surface and pool at the apex of the dome.

In an embodiment, the direction of the normal to the apex of the dome is orientated vertically downwardly, in use. In this way, condensate liquids can flow along the dome under the force of gravity and pool at the apex, where the optical element is located. The apex of the dome may therefore form a gravitational minimum point on the condensing surface.

In an embodiment, the light source is arranged to emit light toward an illumination surface of the condensing wall, wherein the illumination surface is provided opposite to the condensing surface. More specifically, the illumination surface is provided on the opposite side of the condensing wall to the condensing surface. In this way, the light source can be optically coupled to the waveguide of the optical element if the condensing wall is disposed between the light source and the optical element. In a domed configuration this allows the light source to be conveniently located within the dome. The condensing wall may also be disposed between the detector and the optical element, and in a domed configuration the detector may also be positioned within the dome. This can protect electronics in the light source and the detector from the condensed liquid. In various embodiments, the illumination surface may be provided opposite to the condensing surface at an angle to the surface normal of the condensing surface. Preferably the condensing wall is optically transparent to light from the light source.

In an embodiment, the condensing surface projects from a first side of the device, and the light source and the detector are arranged on a second side of the device. In this way, when the device is installed in a storage container, for example, the condensing surface can project through a hole in a storage container wall. The remainder of the device, including the light source and the detector, can be provided on the outside of the storage container. The device can then be secured to the storage container wall to hold it in place. It is therefore easy to retrofit the device to the storage container. Furthermore, the electronic components of the device, namely the light source, the detector, and the transmitter are disposed exterior to the storage container and are therefore easily accessible should any maintenance need to be performed. The electronic components of the device are also advantageously shielded from the contents of the storage container. Furthermore, the transmitter is better able to transmit the data feed if it is installed exterior to the storage container.

In an embodiment, the detector is configured to detect a change in the wavelength or the position of a resonant guided mode of the plurality of resonant guided modes thereby to detect the presence of microbes in the liquid that pools around the diffraction grating or the optical element. The magnitude of the change in the wavelength or position of the resonating light may be indicative of the degree of increase in the presence of microbes, their metabolic activity and the degree of contamination. A certain number of microbes may initially present within the enclosed environment, and the device is able to detect increases in the quantity and activity of the microbes present.

In an embodiment, the detector is configured to produce a data feed comprising data indicating the wavelength or the resonance position of a resonant guided mode of the plurality of resonant guided modes. Preferably, the device further comprises a transmitter configured to transmit the data feed produced by the detector. In this way, the data feed from the detector can indicate when microbial growth has been detected on the condensing surface of the device. The data can be detected by a computer system that is remote from the enclosed environment. In this way, changes in the condensate composition detected by the sensor can be used as an early warning system to which an appropriate mitigation response can be made by the manufacturing process owner.

Preferably, the device further comprises a battery configured to provide power to the light source, the detector, and the transmitter. In this way, the device can be installed within an enclosed environment, such as a storage tank, prior to the storage tank being filled with material. The device can then measure any microbial or fungal contamination present within the enclosed environment during the course of its use and remotely transmit the data feed produced by the detector to a computer system. The computer system can receive and analyse the data within the data feed.

In another aspect of the present invention there is provided a storage tank, comprising: a liquid medium or a solid medium comprising a liquid medium, located at the base of the tank; and a gaseous medium, in contact with the solid or liquid medium, located at the top of the tank; and a device as described herein, wherein the device is positioned at a top wall of the tank within the gaseous medium so that liquid can condense on the condensing surface.

In some embodiments, a solid medium stored in the tank may be a powder or granulate substance. The powder or granulate substance may comprise liquid that can evaporate therefrom to form a vapour suspended by the gaseous medium within the tank. Vapour within the tank can condense on the condensing surface.

Preferably, the condensing surface of a device as described herein is provided through an aperture in the top wall of the tank. In this way, the device is easily retrofitted into the ceiling of the storage tank. Other components of the device, such as electronic components, are not required to be provided within the tank and are therefore shielded from its contents.

In another aspect of the present invention there is provided a system, comprising: a conduit for a gas; and a device as described herein, wherein the device is installed at an upper wall of the conduit, and wherein the device is positioned within the gas so that liquid can condense on the condensing surface.

Preferably, the condensing surface of a device as described herein is provided through an aperture in the upper wall of the conduit. In this way, the device is easily retrofitted into the upper section of the conduit. Other components of the device, such as electronic components, are not required to be provided within the conduit and are therefore shielded from its contents.

Devices as described herein may be installed in a variety of settings such as air-conditioning units, aircraft fuel systems, pipelines, and food and beverage processing units, mines, showers, bathrooms, washing machines, dishwashers.

In another aspect of the present invention there is provided a method for detecting microbial growth, comprising: obtaining a data feed from a device as described herein, wherein the data feed comprises data indicating a property of a resonant guided mode of the plurality of resonant guided modes ; processing the data feed to observe a change in the property of the resonant guided mode; and determining, based on the observed change in the property of the resonant guided mode, the presence of microbes in the liquid pooled around the optical element.

In another aspect of the present invention there is provided a storage tank, comprising: liquid medium or a solid medium comprising a liquid medium, located at the base of the tank; and a gaseous medium, in contact with the solid or liquid medium, located at the top of the tank; and a device, wherein the device comprises: a light source that is configured to be optically coupled to an optical element, the optical element comprising a waveguide and a diffraction grating, that together support a plurality of guided mode resonances of selected wavelengths of light from the light source; a condensing wall having a condensing surface on which liquid in gas can condense, wherein the optical element is disposed on the condensing surface; and a detector configured to detect a property of a resonant guided mode of the plurality of resonant guided modes thereby to detect the presence or absence of microbes in the liquid that pools around the optical element; wherein the device is positioned at a wall of the tank, such as a top wall, within the gaseous medium so that liquid can condense on the condensing surface and the optical element.

In some embodiments, a solid medium stored in the tank may be a powder or granulate substance. The powder or granulate substance may comprise liquid that can evaporate therefrom to form a vapour suspended by the gaseous medium within the tank. Vapour within the tank can condense on the condensing surface.

Preferably, the condensing surface of the device is provided through an aperture in the top wall of the tank. In this way, the device is easily retrofitted into the ceiling of the storage tank. Other components of the device, such as electronic components, are not required to be provided within the tank and are therefore shielded from its contents.

In another aspect of the present invention there is provided a system, comprising: a conduit for a gas; and a device, wherein the device comprises: a light source that is configured to be optically coupled to an optical element, the optical element comprising a waveguide and a diffraction grating, that together support a plurality of guided mode resonances at selected wavelengths of light from the light source; a condensing wall having a condensing surface on which liquid in gas can condense, wherein the optical element is disposed on the condensing surface; and a detector configured to detect a property of a resonant guided mode of the plurality of resonant guided modes thereby to detect the presence or absence of microbes in the liquid that pools around the optical element; wherein the device is installed within a wall of the conduit, and wherein the device is positioned within the gas so that liquid can condense on the condensing surface.

Preferably, the condensing surface of the device is provided through an aperture in the upper wall of the conduit. In this way, the device is easily retrofitted into the upper section of the conduit. Other components of the device, such as electronic components, are not required to be provided within the conduit and are therefore shielded from its contents.

In another aspect of the present invention there is provided a method of detecting microbial activity, comprising: receiving, on an optical element disposed on a condensing surface of a guided mode resonance sensor, condensed liquids; measuring a property of a resonant guided mode supported by the optical element; and; determining, based on the measured property of the resonant guided mode, the presence of microbes in the condensed liquid on the optical element.

Various features of the embodiments of devices described herein may be implemented as method steps, and vice versa.

### BRIEF DESCRIPTION OF DRAWINGS

Aspects of the present invention will now be described by way of example, by reference to the drawings, in which:
Figure IA is a flow diagram of exemplary stages of contamination in a storage container;
Figure 1B is a flow diagram of exemplary stages of contamination in a storage container;
Figure 2 is a schematic diagram of a guided mode resonance sensor in an embodiment of the invention;
Figure 3 is a diagram of a condensing wall in an embodiment of the invention;
Figure 4 is a schematic diagram of a condensing wall in an embodiment of the invention;
Figure 5 is a diagram of a device for detecting microbial activity in an embodiment of the invention;
Figure 6 is a diagram of a liquid storage tank in an embodiment of the invention; and
Figure 7 is a system comprising a conduit for a gas in an embodiment of the invention.

### DETAILED DESCRIPTION

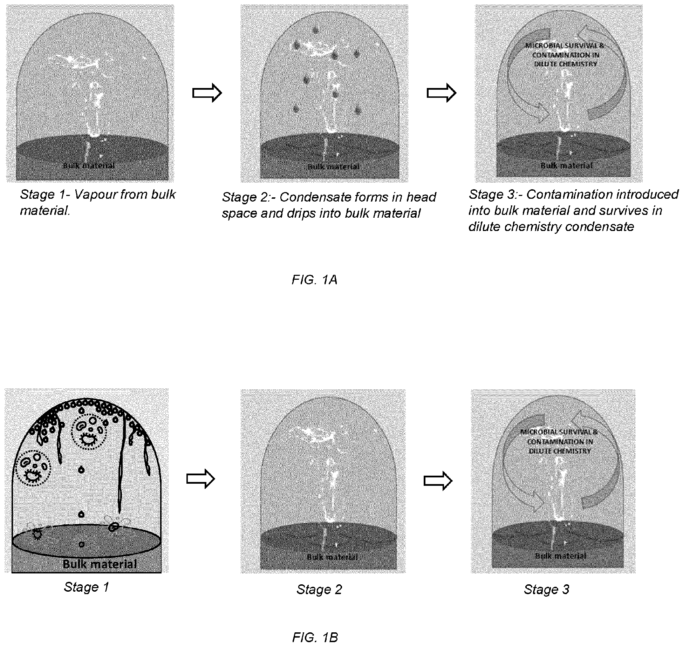

Figure 1A is a flow diagram of three exemplary stages of the process of contamination in a liquid cycle in a storage container.

At stage 1 of the process, a liquid bulk material held within the storage container begins to release moisture into the air through evaporation and splashing, forming a vapour. At stage 2 of the process, microbial growth begins to occur. Some of the microbes are then transported by the vapour, or liquid droplets within the air or gas, in the storage container. At stage 3 of the process, the evaporated bulk material can condense to form droplets on the walls and the ceiling of the storage container, thereby depositing the microbes onto the walls and the ceiling. These droplets containing the microbes can then flow down the walls of the container and back into liquid bulk material. This cycle can continue, at a pace that is determined by the temperature of the media within the container, spreading microbes around the surfaces of the storage container. Contaminating matter can be introduced into the bulk material and can survive in dilute chemistry condensate.

Figure 1B is a flow diagram of three exemplary stages of the process of contamination in a liquid cycle in a storage container.

At stage 1 of the process, vapour or moisture in the air or gas can condense onto surfaces inside the container due to changes in temperature, forming droplets. For example, onto walls or the ceiling of the storage container, or onto the surface of a bulk material stored therein. Some microbes may be present on surfaces within the storage container as it may not have been fully or at least partially sterilised. Alternatively, or in addition, microbes may be present in the vapour or the air or gas that is initially enclosed within the container. At stage 2, the droplets of condensation can carry the microbes into the bulk material due to a liquid flow along the container walls. At stage 3, the microbes continue to grow where they have been deposited, leading to contamination of other areas of the container.

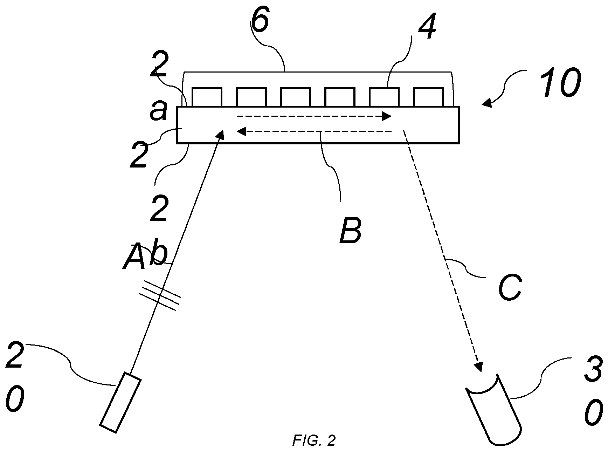

Figure 2 is a schematic diagram of a guided mode resonance sensor in an embodiment of the invention. The guided mode resonance sensor comprises an optical element 10, a light source 20, and a detector 30. The optical element 10 comprises a waveguide 2 and a plurality of individual grating elements 4 which collectively form a diffraction grating. The grating elements 4 are disposed on an upper surface 2a of the waveguide 2. A liquid film 6 is disposed on the diffraction grating.

The optical element 10 is illuminated by incident light A emitted from the light source 20. The incident light A from the light source 20 is broadband and couples to the waveguide 2 at a lower surface 2b. Some of the incident light A diffracts, and particular diffracted wavelengths of the incident light A are captured within the waveguide 2 as resonating light B. The optical element 10 therefore supports a plurality of guided mode resonances at selected wavelengths of light from the light source 20. Some of the resonating light B subsequently couples out of the waveguide at the lower surface 2b. This output light C is narrowband and can be detected by the detector. The wavelength of the output light C is dependent on the properties of the diffraction grating and the effective refractive index of the optical element 10 and the liquid film 6. By measuring the wavelength of the output light C, the effective refractive index of the optical element 10 and the liquid film 6 can be calculated.

If a change in the chemistry of the liquid film 6 occurs, for example if microbes begin to grow, the wavelength of the resonating light B will shift, causing a shift in the wavelength of the output light C. Such changes in chemistry could include the formation or presence of proteins, polysaccharides, the growth of microbes, and metabolic activity associated with these.

Microbes, particularly bacteria, can excrete and secrete substances into the liquid film 6. These substances will change properties of the resonating light B, or the resonant guided modes. The guided mode resonance sensor can therefore indirectly detect the presence of microbes based on the refractive index of the biofilm 6.

The light source 20 is a broadband light source that emits light A of a plurality of different wavelengths. In this embodiment, the light source 20 is orientated to emit light A such that it is incident on the lower surface 2b of the waveguide 2. The light A is incident on the lower surface 2b at an angle that is between that of the direction of the normal to the lower surface 2b and the direction that is parallel to the lower surface 2b. In alternative embodiments, the light source 20 may be orientated such that the light A is emitted in a direction parallel to the normal to the lower surface 2b.

In this embodiment, the detector 30 is a camera that directly images the output light C that couples out of the waveguide 2. In alternative embodiments, the detector 30 may be another type of photodetector such as an array of photodiodes. The detector 30 is orientated in a direction facing the lower surface 2b and that is parallel to the direction of travel of the output light C.

Described herein is one regime for implementing a guided mode resonance sensor to detect the presence of microbes. The skilled person would understand that there are number of different ways of implementing or arranging the optical element 10, the light source 20, and the detector 30 as described herein in order to detect properties of the liquid film 6, and in particular the growth of microbes. The light source 20 and the detector 30 could be provided in different positions, as is known in the art, to determine the wavelength of the resonating light B.

In various embodiments, the light source 20 may instead be a narrowband light source, or a broadband light source provided with a narrowband filter, and the diffraction grating may be a chirped diffraction grating. In such embodiments, the detector 30 may be configured to detect a position of the resonating light B (the resonant guided modes). The observed position of the output light C is dependent on the properties of the chirped diffraction grating and the effective refractive index of the optical element 10 and the liquid film 6. By measuring the position of the output light C, the effective refractive index of the optical element 10 and the liquid film 6 can be calculated. If a change in the chemistry of the liquid film 6 occurs, for example if microbes begin to grow, the observed position of the resonating light B will shift, causing a shift in the position of the output light C. The term "position" can be interpreted as being the location of the point of maximum intensity of the resonating or output light.

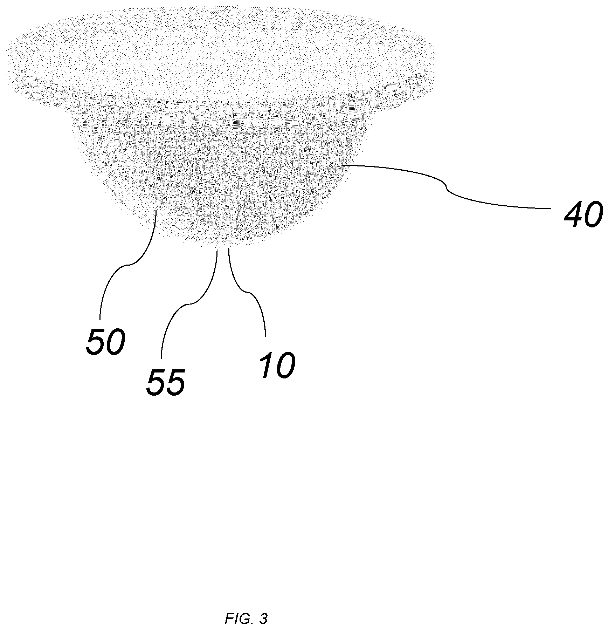

Figure 3 is a diagram of a condensing wall 40 in an embodiment of the invention. The condensing wall 40 comprises a condensing surface 50, which comprises a collecting point 55. An optical element 10 is disposed on the collecting point 55. The optical element 10 may be the optical element 10 described in the embodiment of the invention according to Figure 2. A light source and a detector (not shown) are disposed within the dome of the condensing wall and are arranged with respect to the optical element 10 in the manner described in the embodiment of the invention according to Figure 2. In an alternative embodiment, the light source and the detector may be disposed outside of the dome but arranged in the same manner with respect to the optical element 10.

In a preferred embodiment of the invention, the condensing wall 40 has a hemispherical dome shape and its exterior surface forms the condensing surface 50. The condensing wall 40 is transparent and comprises glass or another optically transparent material. The direction of the normal to the apex of the dome is orientated vertically downwardly in Figure 3 and this is the preferred orientation of use of the condensing wall 40. The collecting point 55 is located at the apex of the dome, which is a stationary point on the collecting surface 50. The optical element 10 is also located at the apex of the dome.

In alternative embodiments, the condensing wall 40 may have a different shape to that of a hemispherical dome. The condensing wall 40 may have another type of curved or convex shape, for example. In alternative embodiments, the collecting point 55 may be located on a lower portion of the condensing surface 50. The lower portion of the condensing surface 50 may surround the apex of the dome. In further alternative embodiments, collecting point 55 may be at any location on the condensing surface 50, depending on the intended orientation of use of the condensing wall 40. The optical element 10 may or not be disposed on the collecting point 55 in these alternative embodiments.

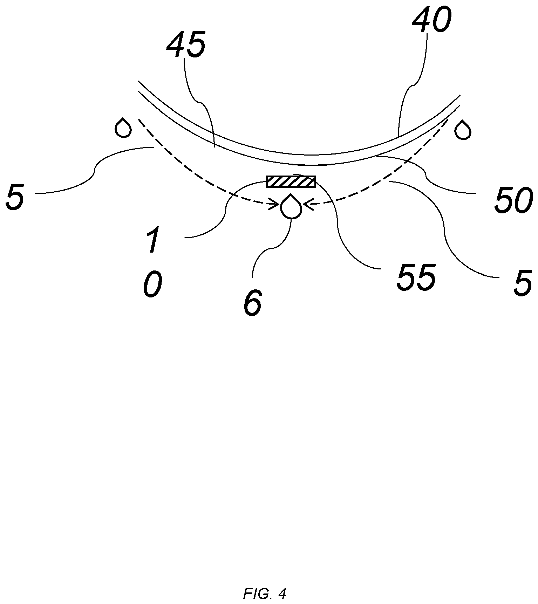

Figure 4 is a schematic diagram of the condensing wall 40 in the embodiment of the invention according to Figure 3. The condensing wall 40 has a hemispherical dome shape and its exterior surface forms a condensing surface 50. The interior surface of the condensing wall forms an illumination surface 45. The condensing surface 50 comprises a collecting point 55 at which an optical element 10 is disposed. The optical element may be the optical element 10 described in the embodiment of the invention according to Figure 2.

As described in relation to stage 3 of the exemplary contamination process of Figure 1A, evaporated materials within a container can condense to form droplets on the walls and the ceiling of the storage container, thereby depositing the microbes onto the walls and the ceiling. As described in relation to stage 3 of the exemplary contamination process of Figure 1B, vapour can condense to form droplets on the walls and the ceiling of the storage container, thereby depositing the microbes onto the walls and the ceiling. Condensed liquids will randomly form at different locations on the condensing surface 50 in such scenarios. In order for microbes within condensed liquids to be detected by a guided mode resonance sensor such as that described in the embodiment according to Figure 2, the condensed liquids are brought into contact with the optical element 10.

As the condensing surface 50 may be dome shaped, it is shaped to support a flow of condensed liquids under gravity along the flow paths 5 towards the collecting point 55 where the liquid can pool around the optical element 10 to form a liquid film 6. Droplets of condensed liquids that form on upper portions of the condensing surface 50, where the gradient of the surface 50 is steeper, will flow along the surface 50 to the collecting point 55 due to the curvature of the surface 50. These droplets may follow a flow path 5 such as that depicted in Figure 4.

At the collecting point 55, droplets of condensed liquids can pool on the optical element 10 forming a film of liquid 6, thereby bringing the condensed liquids into contact with the optical element 10. The surface tension of the liquid film 6 will prevent the condensed liquids from falling away from the optical element 10, at least until the liquid film 6 reaches a critical size. Properties of the liquid film 6 can then be measured using the technique described in the embodiment of the invention according to Figure 2.

Microbes or substances within the film 6 can then be detected using the technique described in the embodiment according to Figure 2. Any change in the wavelength of the light output from the optical element 10 received at the detector can indicate the presence or absence of microbes within the liquid film 6 surrounding the optical element 10.

In various embodiments, to encourage the flow of condensed liquids under gravity towards the collecting point 55, properties of the condensing wall 40 and the condensing surface 50 can be adjusted. These properties include, but are not limited to, the geometry of the condensing surface 50, the geometry and the surface area of the collecting point 55, and the material of the condensing wall 40.

For example, in this embodiment, the geometry of the condensing surface 50 is that of a dome. In alternative embodiments, the geometry of the condensing surface 50 may comprise a conical shape or another suitable shape to promote the flow of condensed liquids under gravity towards a collecting point 55. In various embodiments, the illumination surface 45 may be provided opposite to the condensing surface 50 at an angle to the surface normal of the condensing surface 50.

In this embodiment, the surface area of the collecting point 55 is relatively small compared to that of the condensing surface 50 as it is located at the apex of the dome. In alternative embodiments, the condensing surface 50 may comprise a flat section which forms the collecting point 55 that the optical element 10 can be disposed upon.

In this embodiment, the condensing wall 40 comprises glass. In alternative embodiments, the condensing wall 40 may comprise a different optically transparent material such as Perspex. Various materials may be selected to optimise the wettability, surface energy, topography, and/or hydrophobicity of the condensing surface.

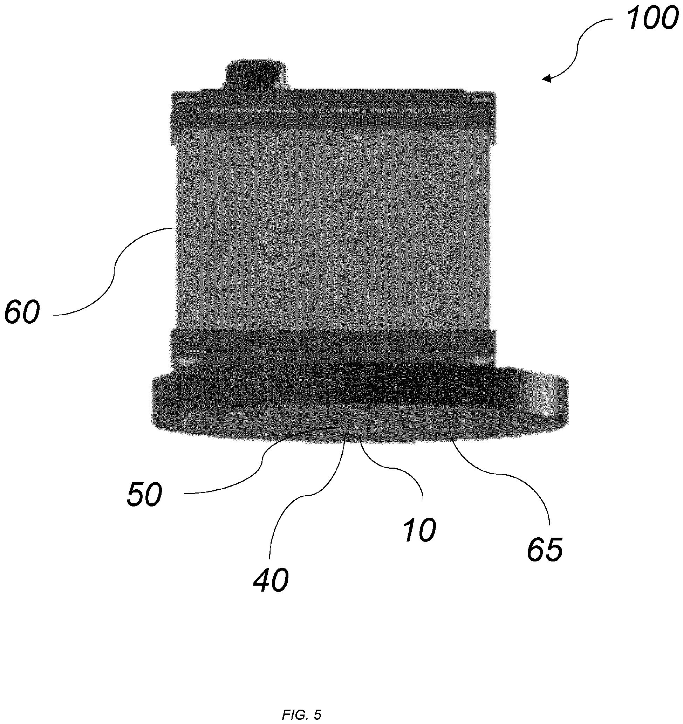

Figure 5 is a diagram of a device 100 for detecting microbial activity in an embodiment of the invention. The device 100 comprises an optical element 10. The optical element may be the optical element 10 described in the embodiment of the invention according to Figure 2. The device 100 further comprises a condensing wall 40 comprising a condensing surface 50 as described in the embodiments of the invention according to Figures 3 and 4. The optical element 10, the condensing wall 40 and the condensing surface 50 are supported by but provided external to a housing 60. The optical element 10, the condensing wall 40 and the condensing surface 50 project from a bottom portion 65, or shielding portion, of the housing 60. The housing 60 also supports a light source, a detector, a transmitter and a battery (all not shown). The light source, the detector, the transmitter and the battery are disposed within the housing, on the other side of the device. The battery is configured to provide power to the light source, the detector, and the transmitter.

In use, the bottom portion 65 of the device is installed in the ceiling, or upper wall, of a storage container or the like. The only components of the device that are provided within the storage container are therefore the optical element 10, the condensing wall 40 and the condensing surface 50. These components can therefore be in fluidic contact with vapour within the storage tank. In this arrangement, the device can be retrofitted installed into an existing storage container by drilling a hole in the ceiling or a wall of the container and securing the bottom portion 65 around or within the hole.

The remaining components of the device, the electronic components, are provided exterior to the storage container in use as these are disposed within the housing 60. The electronic components (the light source, detector, and transmitter) are protected from damage by the contents of the storage container in this arrangement.

The detector produces a data feed which indicates the wavelength or position of the light that is output from the optical element 10. Any change in the wavelength or position of the light output from the optical element 10 thereby indicates the presence of condensed liquids on the optical element and microbes. The data feed is fed to the transmitter either by a wired or wireless connection. The transmitter then sends the data feed to a computer system or another device that interprets or analyses the data within the data feed. The transmitter may send the data feed using a wired or wireless connection.

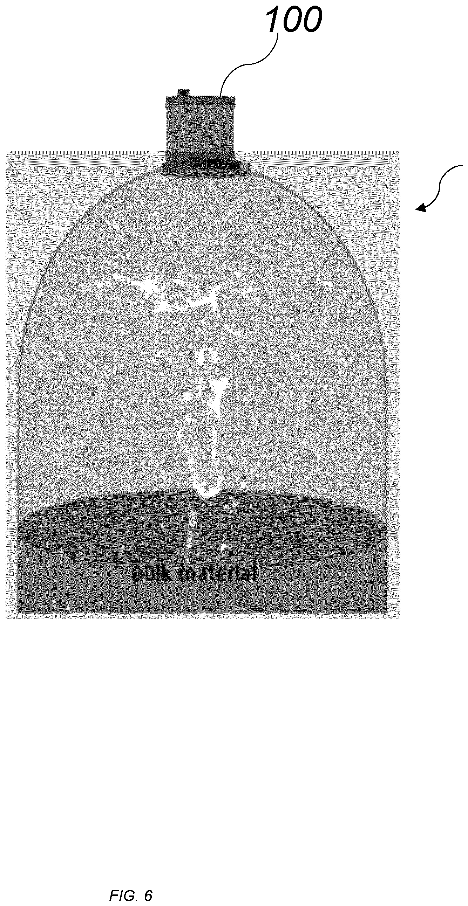

Figure 6 is a diagram of a storage tank 600 in an embodiment of the invention. A device 100 for detecting microbial activity is provided through, and is secured to, an aperture in the top wall of the tank. More specifically, the condensing surface of the device is provided through an aperture in the top wall of the tank and is disposed within the storage tank 600. The device 100 may be the device described in the embodiment of the invention according to Figure 5. Alternatively, the device 100 may be another device comprising a guided mode resonance sensor with a condensing surface and an optical element disposed thereon.

The storage tank 600 comprises a liquid bulk material, or a solid bulk material that contains moisture, that is susceptible to evaporation under storage conditions. The solid or liquid bulk material is located at the base of the tank. A gaseous medium, in this case vapour, is present at the top of the tank and is in contact with the solid liquid bulk material and a condensing surface of the device, which has an optical element disposed upon it. The device is positioned within the moist air so that the moist air can condense on the condensing surface and the optical element.

The shape of the condensing surface can vary in different embodiments. In some embodiments, such as in pressured containers, it may be desirable to provide a condensing surface that is substantially flat so that it is parallel, in use, with the surfaces of the storage tank. The liquid storage tank 600 typically includes a fill line or a fill indicator, which is a maximum fill level for liquid. The device 100 is positioned above the fill line so that it is disposed in the gaseous medium so that liquid in the vapour can condense on the condensing surface.

In alternative embodiments, the device 100 may be fully disposed within the storage tank 600. In further alternative embodiments, the device 100 may installed on or in a side wall of the storage tank 600 above the surface level of the solid liquid contained therein. In alternative embodiments, the liquid storage tank 600 may comprise a powder or a granulate instead of a liquid. The powder or granulate may comprise trace quantities of liquid that can evaporate to form a vapour within the storage tank.

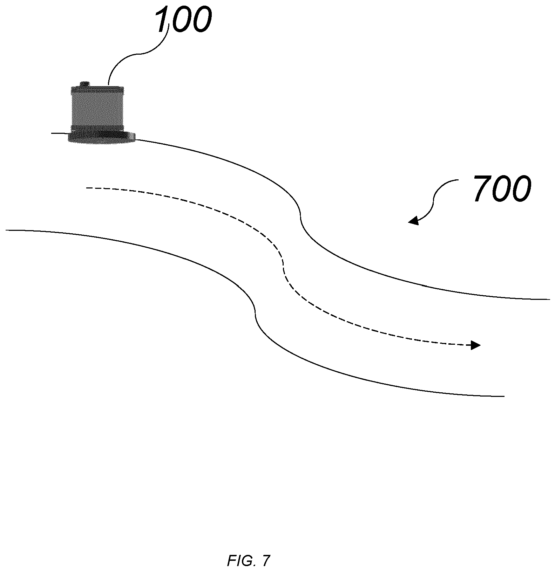

Figure 7 is a system comprising a conduit 700 for a gas in an embodiment of the invention. The conduit may form part of an air conditioning unit or an industrial chemical processing plant, for example. A device 100 for detecting microbial activity is provided through, and is secured to, an aperture in an upper wall of the conduit 700. The device 100 may be the device described in the embodiment of the invention according to Figure 5. Alternatively, the device 100 may be another device comprising a guided mode resonance sensor with a condensing surface and an optical element disposed thereon. As explained above, the condensing surface may be substantially flat in this situation, and this may be particularly advantageous in pressurised conduits. In this situation, the condensing surface may be substantially parallel with the internal surfaces of the conduit 700.

The conduit 700 comprises a gas that comprises moisture that susceptible to condensation when flowing through the conduit 700. The device 100 is positioned within the gas so that liquid can condense on a condensing surface of the device. An optical element is disposed on the condensing surface and therefore moisture can also condense on the optical element.

Devices as described herein may be installed in a variety of settings such as air-conditioning units, aircraft fuel systems, hydrocarbons pipelines, and food and beverage processes, mines, showers, bathrooms, washing machines, and dishwashers.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

## Claims

1. A device for detecting microbial activity, comprising:
a light source that is configured to be optically coupled to an optical element, the optical element comprising a waveguide and a diffraction grating, that together support a plurality of resonant guided modes at selected wavelengths of light from the light source;
a condensing wall having a condensing surface on which liquid in gas can condense, wherein the condensing surface is shaped to support the flow of condensed liquids under gravity towards a collecting point where the liquid can pool around the optical element; and
a detector configured to detect a property of a resonant guided mode of the plurality of resonant guided modes thereby to detect the presence or absence of microbes in the liquid that pools around the optical element.

2. A device according to claim 1, wherein the condensing surface includes a lower portion where the collecting point is located.

3. A device according to claim 1 or claim 2, wherein the condensing surface has a domed shape.

4. A device according to claim 3, wherein the optical element is disposed at the apex of the dome shaped condensing surface.

5. A device according to claim 4, wherein the direction of the normal to the apex of the dome is orientated vertically downwardly, in use.

6. A device according to any preceding claim, wherein the light source is arranged to emit light toward an illumination surface of the condensing wall, wherein the illumination surface is provided opposite to the condensing surface at an angle to the surface normal of the condensing surface.

7. A device according to any preceding claim, wherein the light source and the detector are arranged on the opposite side of the condensing wall to the condensing surface.

8. A device according to claim 7, wherein the condensing wall has a domed shape, and wherein the light source and the detector are arranged within the dome shaped condensing wall.

9. A device according to any preceding claim, wherein the condensing surface projects from a first side of the device, and wherein the light source and the detector are arranged on a second side of the device.

10. A device according to any preceding claim, wherein the detector is configured to detect a change in the wavelength or the position of a resonant guided mode of the plurality of resonant guided modes thereby to detect the presence of microbes in the liquid that pools around the diffraction grating.

11. A device according to any preceding claim, wherein the detector is configured to produce a data feed comprising data indicating the wavelength or position of a resonant guided mode of the plurality of resonant guided modes.

12. A device according to claim 11, wherein the device further comprises a transmitter configured to transmit the data feed produced by the detector.

13. A device according to claim 12, wherein the device further comprises a battery configured to power the light source, the detector, and the transmitter.

14. A storage tank, comprising:
a liquid medium or a solid medium comprising a liquid medium, located at the base of the tank.
and a gaseous medium, in contact with the solid or liquid medium, located at the top of the tank; and
the device of any of the preceding claims, wherein the device is positioned at a top wall of the tank within the gaseous medium so that liquid can condense on the condensing surface.

15. A storage tank according to claim 14, wherein the condensing surface of the device of any of claims 1 to 13 is provided through an aperture in the top wall of the tank.

16. A system, comprising:
a conduit for a gas; and
the device of any of claims 1 to 13, wherein the device is installed at an upper wall of the conduit, and wherein the device is positioned within the gas so that liquid can condense on the condensing surface.

17. A system according to claim 16, wherein the condensing surface of the device of any of claims 1 to 13 is provided through an aperture in the upper wall of the conduit.

18. A method of detecting microbial activity, comprising:
obtaining a data feed from the device of any of claim 1 to 13 wherein the data feed comprises data indicating a property of a resonant guided mode of the plurality of resonant guided modes;
processing the data feed to observe a change in the property of the resonant guided mode; and
determining, based on the observed change in the property of the resonant guided mode, the presence of microbes in the liquid pooled around the optical element.

19. A storage tank, comprising:
a liquid medium or a solid medium comprising a liquid medium, located at the base of the tank; and
a gaseous medium, in contact with the solid or liquid medium, located at the top of the tank; and
a device, wherein the device comprises:
a light source that is configured to be optically coupled to an optical element, the optical element comprising a waveguide and a diffraction grating, that together support a plurality of guided mode resonances at selected wavelengths of light from the light source;
a condensing wall having a condensing surface on which liquid in gas can condense, wherein the optical element is disposed on the condensing surface; and
a detector configured to detect a property of a resonant guided mode of the plurality of resonant guided modes thereby to detect the presence or absence of microbes in the liquid that pools around the optical element.

20. A liquid storage tank according to claim 19, wherein the device is positioned at a wall of the tank within the gaseous medium so that liquid can condense on the condensing surface and the optical element.

21. A system, comprising:
a conduit for a gas; and
a device, wherein the device comprises:
a light source that is configured to be optically coupled to an optical element, the optical element comprising a waveguide and a diffraction grating, that together support a plurality of guided mode resonances at selected wavelengths of light from the light source;
a condensing wall having a condensing surface on which liquid in gas can condense, wherein the optical element is disposed on the condensing surface; and
a detector configured to detect a property of a resonant guided mode of the plurality of resonant guided modes thereby to detect the presence or absence of microbes in the liquid that pools around the optical element.

22. A system according to claim 21, wherein the device is installed at an upper wall of the conduit, and wherein the device is positioned within the gas so that liquid can condense on the condensing surface.

23. A system according to claim 22, wherein the condensing surface of the device is provided through an aperture in the upper wall of the conduit.

24. A method of detecting microbial activity, comprising:
receiving, on an optical element disposed on a condensing surface of a guided mode resonance sensor, condensed liquids;
measuring a property of a resonant guided mode supported by the optical element; and;
determining, based on the measured property of the resonant guided mode, the presence of microbes in the condensed liquid on the optical element.
